# EUROPEAN PATENT APPLICATION

(11) **EP 3 846 178 A1**
(43) Date of publication of application: **07.07.2021**
(21) Application number: 20152162.2
(22) Date of filing: 16.01.2020
(51) Int. Cl.: G16H 40/60, G16H 50/20

(54) **ADAPATIVE MEDICAL VENTILATION MONITORING VIA DEPTH OF SEDATION**

(30) Priority: 31.12.2019 US 201962955578 P
(71) Applicant: Koninklijke Philips N.V., 5656 AG Eindhoven (NL)
(72) Inventor: GHOSH, Erina, 5656 AE Eindhoven (NL); BUIZZA, Roberto, 5656 AE Eindhoven (NL)
(74) Representative: Steenbeek, Leonardus Johannes

(57) **Abstract**

Various embodiments of the present disclosure encompass an adaptive medical ventilation system employing a ventilator (30) controlling a patient ventilation, and an adaptive ventilator monitor (40) adapting the control of the patient ventilation by the ventilator (30) to a patient sedation depth associated with the patient ventilation and to a patient ventilation response to the patient ventilation. To adapt the control of the patient ventilation by the ventilator (30), the adaptive ventilator monitor (40) temporally monitors the patient sedation depth and the patient ventilation response, and derives adaptive ventilation adjustment(s) (e.g., ventilation modes adjustment(s) and/or ventilation setting adjustment(s)) from a cyclic adaptation of the patient ventilation to the temporal monitoring of the patient sedation depth and the patient ventilation response.

## Description

### FIELD OF THE INVENTION

The present disclosure generally relates to mechanical ventilations for medical purposes. The present disclosure specifically relates to a personalization of ventilation settings and/or ventilation modes to support the patient breathing naturally whereby the patient may be weaned off the ventilator.

### BACKGROUND OF THE INVENTION

A large number of patients in intensive care units (ICU) require ventilation support and therefore are mechanically ventilated. While mechanical ventilation supports the respiratory needs of the patient during acute illness, minimizing the duration of mechanical ventilation is important in the recovery process. More particularly, a longer duration of mechanical ventilation beyond the patient's acute illness has been associated with an increased length of patient stay in ICU, increased risk of patient mortality and poorer outcomes for the patient post- hospital discharge. Therefore, the goal is to minimize the duration of ventilation to thereby facilitate a change to less invasive modes of ventilation and more supportive ventilation strategies, which allow the patient to breathe on their own.

To implement this goal, clinicians do spontaneous breathing trials daily to check if the patient can be extubated. For example, in a spontaneous breathing trial, the ventilator is set to a minimum pressure support able to compensate the tubing resistance and minimum positive end-expiratory pressure (PEEP). If the patient can breathe on his/her own, then that patient is extubated.

Current clinical guidelines propose a daily schedule of spontaneous breathing trial if the patient meets set criteria. As such, clinicians have been doing spontaneous breathing trials daily or as indicated. However, the process doesn't account for the patient's depth of sedation, thus failing to factor in a degree a patient's pharmacodynamical level and/or a degree a patient's pharmacodynamical level effects the ventilation. Specifically, in the context of ventilation, pharmacokinetics describes a sedation drug concentration-time courses in body fluids resulting from administration of a certain sedation drug dose, and pharmacodynamics describes an observed effect resulting from a certain sedation drug concentration. By failing to account for the patient's depth of sedation, the patient is weaned on a fixed schedule, which may potentially increase the duration of the ventilation, particularly in view of an impersonal transition from a mandatory mode of ventilation (e.g., assisted/control) providing breathing assistance to a spontaneous mode of ventilation supporting possible extubation.

### SUMMARY OF THE INVENTION

The present disclosure describes adaptive medical ventilation monitoring that is applicable to numerous and various medical ventilation applications. The invention is defined by the independent claims. The dependent claims define advantageous embodiments.

The adaptive medical ventilation monitoring of the present disclosure takes a patient's depth of sedation into account for extubation evaluation purposes (i.e., a degree a patient's pharmacodynamical level and/or a patient's pharmacodynamical level effects the ventilation) to thereby provide for an automatic start of a weaning process when the patient is ready to wean instead of weaning the patient on a fixed schedule to thereby facilitate a personal transition from a mandatory mode of ventilation (e.g., assisted/control) providing breathing assistance to a spontaneous mode of ventilation supporting possible extubation.

The present disclosure may be embodied as:
(1) an adaptive ventilator monitor of the present disclosure;
(2) an adaptive medical ventilation system incorporating the adaptive ventilator monitor of the present disclosure; and
(3) an adaptive medical ventilation method utilizing the adaptive ventilator monitor of the present disclosure.

Various adaptive ventilator monitor embodiments of the present disclosure encompass an adaptive ventilator monitor adapting a patient ventilation controlled by a ventilator to a patient sedation depth associated with the patient ventilation and to a patient ventilation response to the patient ventilation.

In operation, the adaptive ventilator monitor temporally monitors the patient sedation depth and the patient ventilation response, and derives adaptive ventilation adjustment(s) from a cyclic adaptation of the patient ventilation to the patient respiration and the patient sedation depth.

The adaptive ventilation adjustment(s) may include ventilation mode adjustment(s) to a ventilation mode of the ventilator, such as, for example, a mode transition between a volume assisted/controlled ventilation mode, a pressure assisted/controlled ventilation mode, volume synchronized intermittent ventilation mode, a pressure synchronized intermittent ventilation mode, a volume spontaneous ventilation mode and a pressure spontaneous ventilation mode.

The adaptive ventilation adjustment(s) may include ventilation setting adjustment(s) to ventilation setting(s) of the ventilator, such as, for example, an increment or a decrement to an oxygen % ventilation setting, an increment or a decrement to a respiratory rate ventilation setting, an increment or a decrement to a tidal volume ventilation setting, an increment or a decrement to an inhaled tidal volume ventilation setting, an increment or a decrement to an exhaled tidal volume ventilation setting, an increment or a decrement to a positive end-expiratory pressure ventilation setting, an increment or a decrement to a trigger sensitivity ventilation setting and/or an increment or a decrement to an inspiratory time ventilation setting.

Various adaptive medical ventilation systems embodiments of the present disclosure encompass a ventilator controlling a patient ventilation, and an adaptive ventilator adapting a patient ventilation controlled by a ventilator to a patient ventilation response to the patient ventilation and to a patient sedation depth associated with the patient ventilation. In operation, the adaptive ventilator monitor temporally monitors the patient sedation depth and the patient ventilation response, and derives adaptive ventilation adjustment(s) from a cyclic adaptation of the patient ventilation to the patient sedation depth and the patient ventilation response.

Again, the adaptive ventilation adjustment(s) may include ventilation mode adjustment(s) to a ventilation mode of the ventilator and/or ventilation setting adjustment(s) to ventilation setting(s) of the ventilator.

Various adaptive medical ventilation methods embodiments of the present disclosure encompass a ventilator controlling a patient ventilation, and an adaptive ventilator adapting a patient ventilation controlled by the ventilator to a patient ventilation response to the patient ventilation and to a patient sedation depth associated with the patient ventilation. The adaptive medical ventilation methods involve temporally monitoring, via the adaptive ventilator monitor, the patient ventilation response to the patient ventilation and the patient sedation depth associated with the patient ventilation, and deriving, via the adaptive ventilator monitor, adaptive ventilation adjustment(s) from a cyclic adaptation of the patient ventilation to the patient ventilation response to the patient ventilation and the patient sedation depth associated with the patient ventilation.

Again, the adaptive ventilation adjustment(s) may include ventilation mode adjustment(s) to a ventilation mode of the ventilator and/or ventilation setting adjustment(s) to ventilation setting(s) of the ventilator.

For purposes of the description and claims of the present disclosure:
(1) terms of the art including, but not limited to, "ventilator", "medical ventilator", "ventilation (and tenses thereof)", "ventilation mode(s)", "ventilation setting(s)", "monitor (and tenses thereof)", "respiration (and tenses thereof)" and sedation (and tenses thereof)" are to be interpreted as known in the art of the present disclosure and as exemplary described in the present disclosure;
(2) more particularly, the term "medical ventilator" broadly encompasses all mechanical ventilators, as known in the art of the present disclosure and hereinafter conceived, for ventilating a patient for any medical reason;
(3) the term "patient ventilation" broadly encompasses any type of ventilation to a patient intubated to a medical ventilator;
(4) the term "patient ventilation response" broadly encompasses any effect of a patient ventilation on a patient respiratory system;
(5) the term "patient sedation depth" broadly encompasses an estimation (e.g., a prediction or a classification) of a degree a patient's pharmacokinetical level effect(s) the patient ventilation and/or of a degree a patient's pharmacodynamical level effect(s) the patient ventilation;
(6) the term "temporal monitoring (and tenses thereof)" broadly encompasses a continual, periodic or intermittent monitoring of the patient sedation depth and the patient ventilation response;
(7) the term "adaptive ventilation adjustment" broadly encompasses any adjustment to a control of a patient ventilation by a medical ventilator;
(8) the term "ventilation mode adjustment" broadly encompasses an adjustment of a current ventilation mode to a different ventilation mode (e.g., adjustment of an assist/control ventilation mode to a spontaneous ventilation mode, or vice-versa) and an adjustment of a current parameterization of a current ventilation mode to a different parameterization of the current ventilation mode (e.g., adjustment from a volume breathe delivery of an assist/control ventilation mode to a pressure breather delivery of the assist/control ventilation mode);
(9) the term "ventilation setting adjustment" broadly encompasses an adjustment of a current ventilation setting to a different ventilation setting in terms of a different numerical value (e.g., adjustment of a current respiratory rate of 10 breaths/minute to 11 breaths/minute) or in terms of a numerical value adjustment (e.g., an increment of 1 breath/minute to a current respiratory rate of 10 breaths/minute, or an decrement of 1 breath/minute to a current respiratory rate of 10 breaths/minute).
(10) the term "cyclic adaptation" broadly encompasses a factoring of the patient sedation depth and the patient ventilation response into the control of the patient ventilation by the medical ventilator on a patient respiration cycle basis (e.g., every patient respiration cycle, every nth patient respiration cycle, each patient respiration cycle of a particular ventilation mode and each nth patient respiration cycle of a particular ventilation mode);
(11) the term "patient sedation scale" broadly encompasses any sedation scale, as known in the art of the present disclosure and hereinafter conceived, of a range of patient sedation scores used to measure an agitation/sedation level of a patient. Examples of a patient sedation scale include, but are not limited to, Richmond Agitation Sedation Scale (RASS) and Ramsey Sedation Scale (RSS);
(12) the term "patient extubatable spontaneous breathing" broadly encompasses a spontaneous breathing by a patient ventilation requiring minimal or zero ventilation support;
(13) more particularly, the term "adaptive medical ventilator monitor" broadly encompasses all structural configurations, as understood in the art of the present disclosure and as exemplary described in the present disclosure, having main circuit board(s) and/or integrated circuit(s) for controlling an application of various principles of the present disclosure for adapting a patient ventilation controlled by a medical ventilator to a patient ventilation response to the patient ventilation and to a patient sedation depth associated with the patient ventilation in accordance with the present disclosure;
(14) the term "application module" broadly encompasses an application incorporated within or accessible by a medical ventilator or a monitor consisting of an electronic circuit (e.g., electronic components and/or hardware) and/or an executable program (e.g., executable software stored on non-transitory computer readable medium(s) and/or firmware) for executing a specific application associated with for adapting a patient ventilation controlled by a medical ventilator to a patient ventilation response to the patient ventilation and to a patient sedation depth associated with the patient ventilation in accordance with the present disclosure; and
(15) the terms "signal", "data" and "command" broadly encompasses all forms of a detectable physical quantity or impulse (e.g., voltage, current, or magnetic field strength) as understood in the art of the present disclosure and as exemplary described in the present disclosure for transmitting information and/or instructions in support of applying various inventive principles of the present disclosure as subsequently described in the present disclosure. Signal/data/command communication various components of the present disclosure may involve any communication method as known in the art of the present disclosure including, but not limited to, signal/data/command transmission/reception over any type of wired or wireless datalink and a reading of signal/data/commands uploaded to a computer-usable/computer readable storage medium.

The foregoing embodiments and other embodiments of the present disclosure as well as various structures and advantages of the present disclosure will become further apparent from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates a first exemplary embodiment of an adaptive medical ventilation system in accordance with the present disclosure;
FIG. 2 illustrates a first exemplary embodiment of the adaptive medical ventilator monitor in accordance with the present disclosure;
FIG. 3 illustrates an exemplary embodiment of a flowchart representative of an adaptive medical ventilation method in accordance with the present disclosure;
FIG. 4 illustrates an exemplary embodiment of the adaptive medical ventilator monitor of FIG. 2 in accordance with the present disclosure;
FIG. 5 illustrates an exemplary embodiment of a flowchart representative of an adaptive medical ventilation monitoring method in accordance with the present disclosure;
FIG. 6 illustrates an exemplary embodiment of a sedation depth estimator of FIG. 4 in accordance with the present disclosure;
FIG. 7 illustrates an exemplary embodiment of a flowchart representative of a sedation depth estimation method in accordance with the present disclosure;
FIG. 8 illustrates an exemplary embodiment of an adaptive ventilation controller of FIG. 4 in accordance with the present disclosure;
FIG. 9 illustrates an exemplary embodiment of a flowchart representative of an adaptive ventilation control method in accordance with the present disclosure;
FIG. 10 illustrates an exemplary adaptive medical ventilation in accordance with the present disclosure; and
FIG. 11 illustrates an exemplary embodiment of the adaptive medical ventilator monitor of FIG. 4 in accordance with the present disclosure.

### DETAILED DESCRIPTION OF THE PREFERRED EMBODIMENTS

The present disclosure is applicable to numerous and various mechanical ventilators/ventilations for any medical purpose. The present disclosure improves upon the prior art by providing adaptive ventilation monitoring monitors, systems and methods premised on factoring a patient sedation depth into a control of a patient ventilation by a medical ventilator.

The adaptive ventilation monitoring monitors, systems and methods of the present disclosure may exclusively factor the patient sedation depth into the control of the patient ventilation by the medical ventilator for purposes of optimizing an ongoing patient ventilation.

The adaptive ventilation monitoring monitors, systems and methods of the present disclosure may additionally factor a patient respiratory response in conjunction with the patient sedation depth into the control of the patient ventilation by the medical ventilator for purposes of optimizing an occurrence of an extubation of the patient ventilation.

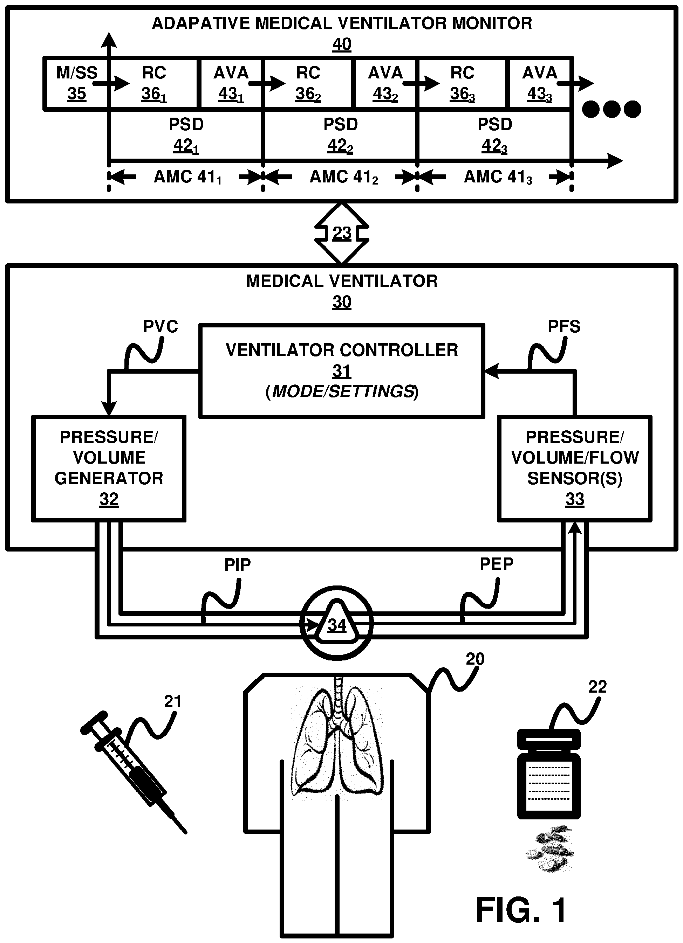

To facilitate an understanding of the present disclosure, the following description of FIG. 1 teaches exemplary embodiments of an adaptive medical ventilation system in accordance with the present disclosure. From the description of FIG. 1, those having ordinary skill in the art of the present disclosure will appreciate how to apply the present disclosure to make and use additional embodiments of adaptive medical ventilation systems in accordance with the present disclosure.

Referring to FIG. 1, a medical ventilator 30 is assisting an intubated patient 20 to breathe (ventilate) as patient 20 is recovering from a surgery or a critical illness, or cannot breathe on his/her own for any reason. Patient 20 is connected to medical ventilator 30 via a ventilation mask 34 and will maintain the connection to medical ventilator 30 until patient 20 is extubated based on patient 20 being able to breathe on his/her own.

As known in the art of the present disclosure, medical ventilator 30 establishes a respiration cycle consisting of a patient inhalation passage PIP between a pressure/volume generator 32 and ventilation mask 34, a patient expiration passage PEP between ventilation mask 34 and pressure/volume/flow sensor(s) 33, a communication of pressure/volume/flow signals PFS between pressure/volume/flow sensor(s) 33 and a ventilation controller 31, and a communication of pressure/volume commands PVC between ventilation controller 31 and pressure/volume generator 32.

More particularly, ventilation controller 31 controls various operating parameters of pressure/volume generator 32 in view of the pressure/volume/flow signals PSF.

Examples of such operating parameters of pressure/volume generator 32 included, but are not limited to, adjustable ventilation modes and adjustable ventilation settings.

Examples of adjustable ventilation modes as known in the art of the present disclosure include, but are not limited to, a volume assist/control ventilation mode, a pressure assist/control ventilation mode, a volume synchronized intermittent mandatory ventilation mode, a pressure synchronized intermittent mandatory ventilation mode, a volume spontaneous ventilation mode and a pressure spontaneous ventilation mode.

Examples of adjustable ventilation settings include, but are not limited to, an adjustable oxygen % ventilation setting, an adjustable respiratory rate ventilation setting, an adjustable tidal volume ventilation setting, an adjustable inhaled tidal volume ventilation setting, an adjustable exhaled tidal volume ventilation setting, an adjustable positive end-expiratory pressure ventilation setting, an adjustable trigger sensitivity ventilation setting and an adjustable inspiratory time ventilation setting.

Prior to and/or during ventilation, patient 20 is sedated, such as, for example, by a needle 21 and/or pills 22. To facilitate the ventilation, the sedation involves a depression of the awareness by patient 20 to the environment and a reduction of the responsiveness of patient 20 to external stimulation on a variety of levels. The lowest level is a minimal sedation that is a drug-induced relief of apprehension with minimal effect on sensorium. The next level is a modern sedation which encompasses a depression of consciousness in which patient 20 may respond to external stimuli (verbal or tactile) whereby airway reflexes, spontaneous ventilation, and cardiovascular function are maintained. The next level is a deep sedation which encompasses a depression of consciousness in which patient 20 cannot be aroused but responds purposefully to repeated or painful stimuli whereby patient 20 may not be able to maintain airway reflexes or spontaneous ventilation, but cardiovascular function is preserved. Further, general anesthesia is a state of unconsciousness whereby the autonomic nervous system of patient 20 is unable to respond to surgical or procedural stimuli.

For each sedation level, the pharmacokinetical level and the pharmacodynamical level of patient 20 effects the ventilation. Specifically, in the context of ventilation, pharmacokinetics describes a sedation drug concentration-time courses in body fluids resulting from administration of a certain sedation drug dose, and pharmacodynamics describes an observed effect resulting from a certain sedation drug concentration. As such, the pharmacokinetical level and the pharmacodynamical level of patient 20 effects a readiness of patient 20 for an attempt to wean off medical ventilator 30.

Instead of attempting to wean patient 20 on a fixed schedule that does not take into account the pharmacokinetical level and the pharmacodynamical level of patient 20, the present disclosure provides an adaptive medical ventilator monitor 40 to adapt the ventilation of patient 20 to a sedation depth of patient 20 for optimizing an ongoing ventilation of patient 20 or to adapt the ventilation of patient 20 to both a sedation depth and a patient ventilation response of patient 20 for optimizing an occurrence of an extubation of patient 20. To this end, adaptive medical ventilator monitor 40 is in communication with ventilator monitor 30 via a communication channel 23, and additionally in communication with either monitoring/sensing/analytical devices/machines generating relevant sedation related data of patient 20 and/or a database/record systems of relevant sedation related data of patient 20.

The sedation depth of patient 20 is an estimation (e.g., a prediction or a classification) of a degree of how the pharmacokinetical level of patient 20 is effecting the ventilation of patient 20 and/or a degree of how the pharmacodynamical level of patient 20 is effecting the ventilation of patient 20. In practice as shown in monitor 40, adaptive medical ventilator monitor 40 temporally monitors a patient sedation depth PSD 42 associated with the ventilation of patient 20 (e.g., a continual, a periodic or an intermittent monitoring), and may derive adaptive ventilation adjustments 43 from a cyclic adaptation of the ventilation of patient 20 to the temporal monitoring of patient sedation depth PSD 42 associated with the ventilation of patient 20.

More particularly, at the onset of adaptive monitoring, a first adaptive monitoring cycle AMC 41₁ involves a temporal monitoring of a patient sedation depth PSD 42₁ concurrently with a temporal monitoring of a respiration cycle RC 36₁ established by an initial ventilation mode and initial ventilation settings M/SS 35.

In practice, the temporal monitoring of the patient sedation depth PSD 42₁ may be continual, periodic or intermittent during adaptive monitoring cycle AMC 41₁, the temporal monitoring of the respiration cycle RC 36₁ may be continual, periodic or intermittent during adaptive monitoring cycle AMC 41₁, and the temporal monitoring of the patient sedation depth PSD 42₁ and of the respiration cycle RC 36₁ may be synchronized or unsynchronized during adaptive monitoring cycle AMC 41₁.

Upon completion of the respiration cycle RC 36₁ and prior to the next respiration cycle RC 36₂, adaptive medical ventilation monitor 40 may derive adaptive ventilation adjustment(s) AVA 43₁ to the initial ventilation mode and/or initial ventilation settings M/SS 35 for the next respiration cycle 36₂. As will be further described in the present disclosure, adaptive ventilator adjustment(s) AVA 43₁ are representative of a degree the pharmacokinetical level of patient 20 is effecting the ventilation of patient 20 and/or a degree the pharmacodynamical level of patient 20 is effecting the ventilation of patient 20 during the adaptive monitoring cycle AMC 41₁ as quantified or qualified by the patient sedation depth PSD 42₁.

A subsequent second adaptive monitoring cycle AMC 41₂ involves a temporal monitoring of a patient sedation depth PSD 42₂ concurrently with a temporal monitoring of a respiration cycle RC 36₂ whereby the initial ventilation mode and/or initial ventilation settings M/SS 35 may have been adjusted by adaptive adjustment(s) AVA 43₁.

In practice, the temporal monitoring of the patient sedation depth PSD 42₂ may be continual, periodic or intermittent during adaptive monitoring cycle AMC 41₂, the temporal monitoring of the respiration cycle RC 36₂ may be continual, periodic or intermittent during adaptive monitoring cycle AMC 41₂, and the temporal monitoring of the patient sedation depth PSD 42₂ and of the respiration cycle RC 362₁ may be synchronized or unsynchronized during adaptive monitoring cycle AMC 41₂.

Upon completion of the respiration cycle RC 36₂ and prior to the next respiration cycle RC 36₃, adaptive medical ventilation monitor 40 may derive additional adaptive ventilation adjustment(s) AVA 43₂ to the ventilation mode and/or initial ventilation settings M/SS 35 for the next respiration cycle 36₃. As will be further described in the present disclosure, adaptive ventilator adjustment(s)AVA 43₂ are representative of a degree the pharmacokinetical level of patient 20 is effecting the ventilation of patient 20 and/or a degree the pharmacodynamical level of patient 20 is effecting the ventilation of patient 20 during the adaptive monitoring cycle AMC 41₂ as quantified or qualified by the patient sedation depth PSD 42₂.

A subsequent third adaptive monitoring cycle AMC 41₃ involves a temporal monitoring of a patient sedation depth PSD 42₃ concurrently with a temporal monitoring of a respiration cycle RC 36₃ established by an initial ventilation mode and initial ventilation setting(s) M/SS 35 as adjusted, if at all, by adaptive adjustment(s) AVA 43₁ and 43₂.

In practice, the temporal monitoring of the patient sedation depth PSD 42₃ may be continual, periodic or intermittent during adaptive monitoring cycle AMC 41₃, the temporal monitoring of the respiration cycle RC 36₃ may be continual, periodic or intermittent during adaptive monitoring cycle AMC 41₃, and the temporal monitoring of the patient sedation depth PSD 42₃ and of the respiration cycle RC 36₃ may be synchronized or unsynchronized during adaptive monitoring cycle AMC 41₃.

Upon completion of the respiration cycle RC 36₃ and prior to the next respiration cycle (not shown), adaptive medical ventilation monitor 40 may derive adaptive ventilation adjustment(s) AVA 43₃ to the initial ventilation mode and/or initial ventilation setting(s) M/SS 35 for the next respiration cycle (not shown). As will be further described in the present disclosure, adaptive ventilator adjustment(s) AVA 43₃ are representative of a degree the pharmacokinetical level of patient 20 is effecting the ventilation of patient 20 and/or a degree the pharmacodynamical level of patient 20 is effecting the ventilation of patient 20 during the adaptive monitoring cycle AMC 41₃ as quantified or qualified by the patient sedation depth PSD 43₃.

Adaptive medical ventilator monitor 40 continues implementing adaptive monitoring cycles 41 until patient 20 is extubated or the monitoring/ventilation is temporally suspended.

Alternatively in practice, during each adaptive monitoring cycles 42, adaptive medical ventilator monitor 40 may derive adaptive ventilation adjustment(s) 43 prior to the temporal monitoring of the respiration cycles 36.

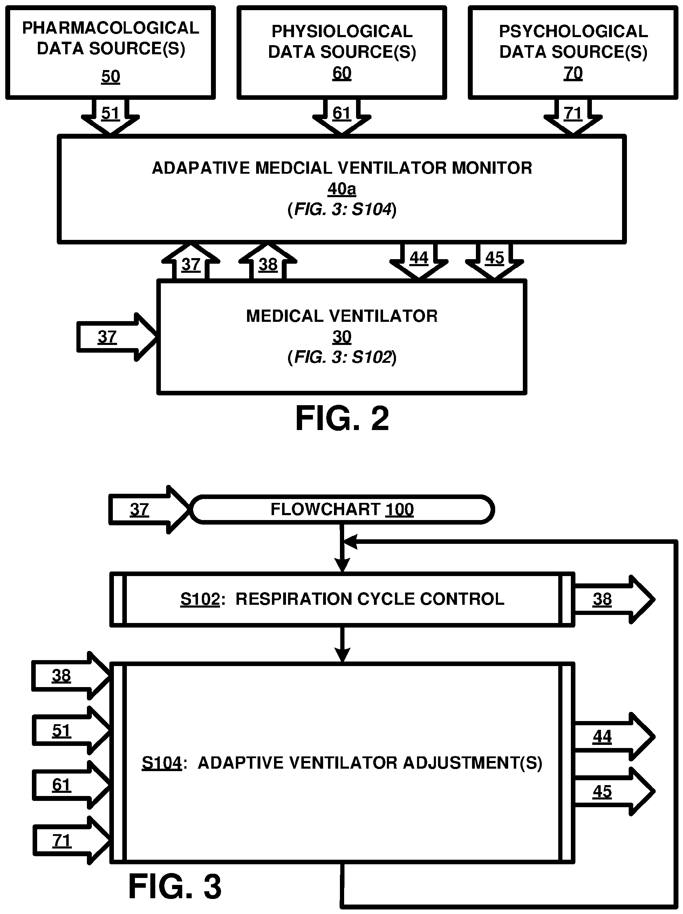

To facilitate a further understanding of the present disclosure, the following description of FIGS. 2 and 3 teaches exemplary embodiments of an adaptive medical ventilation monitor and an adaptive medical ventilation method in accordance with the present disclosure. From the description of FIGS. 2 and 3, those having ordinary skill in the art of the present disclosure will appreciate how to apply the present disclosure to make and use additional embodiments of adaptive medical ventilation monitors and adaptive medical ventilation methods in accordance with the present disclosure.

Referring to FIG. 2, an adaptive medical ventilator monitor 40a of the present disclosure adapts medical ventilator 30 to a patient sedation depth associated with the patient ventilation and a patient respiration response to the patient ventilation. To this end, for estimating the patient sedation depth (e.g., a prediction or a classification of the patient sedation depth), adaptive medical ventilator monitor 40a inputs patient pharmacological data 51 from one or more pharmacological data source(s) 50, inputs patient physiological data 61 from one or more physiological data source(s) 60, and/or inputs patient psychological data 71 from one or more psychological data source(s) 70.

In practice of inputting patient pharmacological data 51, different sedatives and analgesics have different mechanisms of action which may be characterized by pharmacokinetics effects and/or pharmacodynamics effects of the sedatives and analgesics. Patient pharmacological data 51 is informative of sedative drugs given to patient 20 (FIG. 1) (e.g., name of drug, dose, time and mode of administration) to facilitate an estimation of the effective sedative concentration in the patient over time to thereby ascertain the pharmacokinetical effect(s) and/or the pharmacodynamical effect(s) of the sedatives and analgesics on patient 20.

A pharmacological data source 50 may be any source, as known in the art of the present disclosure or herein after conceived, with pharmacologic information of patient 20. Examples of pharmacological data source 50 include, but are not limited, operator manual input of patient pharmacologic information into adaptive medical ventilator monitor 40a or medical ventilator 30, a patient pharmacologic input file generated by a ventilation system and a patient pharmacologic record maintained by an electronic medical record system.

Still referring to FIG. 2, in practice of inputting patient physiological data 61, a physiological status of patient 20 may be an important factor in deciding on whether or not to extubate patient 20. Patient physiological data 61 is informative of a biological functioning of patient 20 to further facilitate an estimation of the effective sedative concentration in patient 20 over time to thereby further ascertain the pharmacokinetical effect(s) and/or the pharmacodynamical effect(s) of the sedatives and analgesics.

A physiological data source 60 may be any source, as known in the art of the present disclosure or herein after conceived, with physiological information of patient 20. Examples of a physiological data source 60 include, but are not limited, a heart rate monitor, a ECG monitor, a blood pressure monitor, a pulse oxygen saturation monitor, a respiratory motion sensor, a patient physiological input file generated by a ventilation system and a patient physiological record maintained by an electronic medical record system.

Still referring to FIG. 2, in practice of inputting psychological data 71, a psychological status of patient 20 may be an important factor in deciding on whether or not to extubate patient 20. Patient psychological data 71 is informative of a level of conscious awareness of patient 20 to further facilitate an estimation of the effective sedative concentration in patient 20 over time to thereby further ascertain the pharmacokinetical effect(s) and the pharmacodynamical effect(s) of the sedatives and analgesics.

A psychological data source 70 may be any source, as known in the art of the present disclosure or herein after conceived, with psychological information of patient 20 informative of a level of conscious awareness of patient 20. Examples of a psychological data source 70 include, but are not limited, a camera for monitoring a body movement of patient 20, a motion sensor for monitoring a body movement of patient 20, a patient psychological input file generated by a ventilation system and a patient psychological record maintained by an electronic medical record system.

Still referring to FIG. 2, medical ventilator 30 and adaptive medical ventilator monitor 40a implement a flowchart 100 representative of an adaptive medical ventilation method of the present disclosure.

Referring to FIGS. 2 and 3, flowchart 100 is initiated upon an operator input of an initial ventilation mode and initial ventilation settings 37 into medical ventilator 30 as known in the art of the present disclosure. Based on the initial ventilation mode and initial ventilation settings 37, medical ventilator 40 controls a first respiration cycle during an initial execution of a stage S102 of flowchart 100 including, as known in the art of the present disclosure, ventilation/breathing (i.e., inhalation/inspiration and exhalation/expiration), pulmonary diffusion (i.e., an exchange of gases between the air in the lungs and blood stream), perfusion (i.e., a transport of gases in the blood) and peripheral diffusion (i.e., an exchange of gases between the blood and tissues).

The first respiration cycle generates respiration status data 38 via pressure/volume/flow sensors 33 (FIG. 1) that are communicated to adaptive ventilator monitor 40a. Respiration status data 38 is informative of including, but not limited to, an oxygenation status and a ventilation status of the patient.

A stage S104 of flowchart 100 encompasses adaptive medical ventilator monitor 40a deriving a ventilation mode adjustment 44 and/or ventilation settings adjustment(s) 45 based on respiration status data 38 and a temporal monitoring of patient pharmacological data 51, patient physiological data 61 and/or patient psychological data 71 during the first respiration cycle of stage S 102 and/or before the second respiration cycle of stage S 104. In practice, as will be further described subsequently in the present disclosure, adaptive medical ventilator monitor 40a estimates a patient sedation depth as a function of patient pharmacological data 51, patient physiological data 61 and/or patient psychological data 71 (e.g., a prediction or a classification), and analyzes the patient respiration response to the ventilation in the context of the estimated patient sedation depth. More particularly, the estimated patient sedation depth will provide an indication of degree a pharmacodynamical level effect(s) and/or a patient pharmacodynamical level effect(s) the oxygenation and the ventilation as indicated by respiration status data 38.

As be further described subsequently in the present disclosure, the analysis of patient respiration response to the ventilation in the context of the estimated patient sedation depth serves as a basis for determining any ventilation mode adjustment 44 and/or ventilation settings adjustment(s) 45 necessary to assist patient 20 toward extubation. Any ventilation mode adjustment 44 and/or ventilation settings adjustment(s) 45 derived by adaptive medical ventilator monitor 40a during stage S104 is communicated to medical ventilator 30 prior to the next execution of stage S102 whereby stage S102 and S104 are sequentially repeated in a loop for each respiratory cycle until patient 20 is extubated or the monitoring/ventilation is temporally suspended.

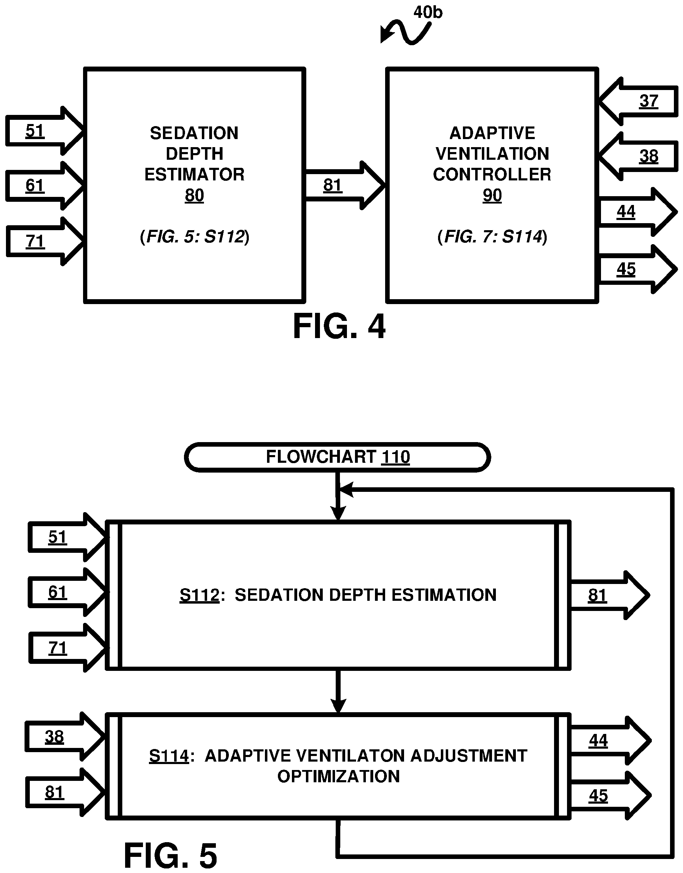

To facilitate a further understanding of the present disclosure, the following description of FIGS. 4 and 5 teaches exemplary embodiments of an adaptive medical ventilator monitor of FIG. 2 and an adaptive medical ventilation method of FIG. 3 in accordance with the present disclosure. From the description of FIGS. 4 and 5, those having ordinary skill in the art of the present disclosure will appreciate how to apply the present disclosure to make and use additional embodiments of adaptive medical ventilator monitors and adaptive medical ventilation methods in accordance with the present disclosure.

FIG. 4 illustrates an embodiment 40b of adaptive medical ventilator monitor 40a (FIG. 2) employing application modules in the form of a sedation depth estimator 80 and an adaptive ventilation controller 90 for implementing a flowchart 110 of FIG. 5 during stage S104 of flowchart 100 (FIG. 3).

Referring to FIGS. 4 and 5, in practice, a stage S112 of flowchart 110 encompasses sedation depth estimator 80 estimating a patient sedation depth 81 as a function of patient pharmacological data 51, patient physiological data 61 and/or patient psychological data 71.

In one exemplary embodiment, sedation depth estimator 80 incorporates a predictive machine learning model, as known in the art of the present disclosure and hereinafter conceived, that is trained to functionally map patient pharmacological data 51, patient physiological data 61 and/or patient psychological data 71 to a patient sedation scale for measuring a quantitative score of the patient sedation depth.

In a second exemplary embodiment, sedation depth estimator 80 incorporates a classification machine learning model, as known in the art of the present disclosure and hereinafter conceived, that is trained to functionally map various patient sedation depth labels/categories (e.g., "extubatable patient sedation depth" and "non- extubatable patient sedation depth") to patient pharmacological data 51, patient physiological data 61 and/or patient psychological data 71 for acquiring a qualitative assessment of the patient sedation depth.

Referring to FIGS. 4 and 5, in practice, a stage S114 of flowchart 110 encompasses adaptive ventilation controller 90 analyzing the patient respiration status data 38 in the context of the estimated patient sedation depth 81 to optimize the ventilation of patient 20 via any necessary ventilation mode adjustment 44 and/or ventilation settings adjustment(s) 45 to drive patient 20 to extubation.

In one exemplary embodiment, adaptive ventilation controller 90 incorporates a reinforcement learning model, as known in the art of the present disclosure and hereinafter conceived, premised on observing the patient ventilation response to ventilation mode adjustment(s) 44 and/or ventilation setting adjustment(s) 45 within the context of the patient sedation depth 81 to thereby ascertain whether an observed sequence of ventilation mode adjustment(s) 44 and/or ventilation setting adjustment(s) 45 is optimizing an occurrence of an extubation of patient 20, which dictates a continuance of the observed sequence of ventilation mode adjustment(s) 44 and/or ventilation setting adjustment(s) 45, or whether the observed sequence of ventilation mode adjustment(s) 44 and/or ventilation setting adjustment(s) 45 is impeding any occurrence of an extubation of patient 20, which dictates a correction to the observed sequence of ventilation mode adjustment(s) 44 and/or ventilation setting adjustment(s) 45.

In a second exemplary embodiment, adaptive ventilation controller 90 incorporates an unsupervised learning model, as known in the art of the present disclosure and hereinafter conceived, trained on patient respiration status 38 and estimated patient sedation depth 81 to thereby identify whether the ventilation mode adjustment(s) 44 and/or ventilation setting adjustment(s) 45 are optimizing an occurrence of an extubation of patient 20, which dictates a continuance of the ventilation mode adjustment(s) 44 and/or ventilation setting adjustment(s) 45 or whether ventilation mode adjustment(s) 44 and/or ventilation setting adjustment(s) 45 are impeding any occurrence of an extubation of patient 20. which dictates a correction to the observed sequence of ventilation mode adjustment(s) 44 and/or ventilation setting adjustment(s) 45.

Stages S112 and S14 are sequentially repeated in a loop for each respiratory cycle until patient 20 is extubated or the monitoring/ventilation is temporally suspended.

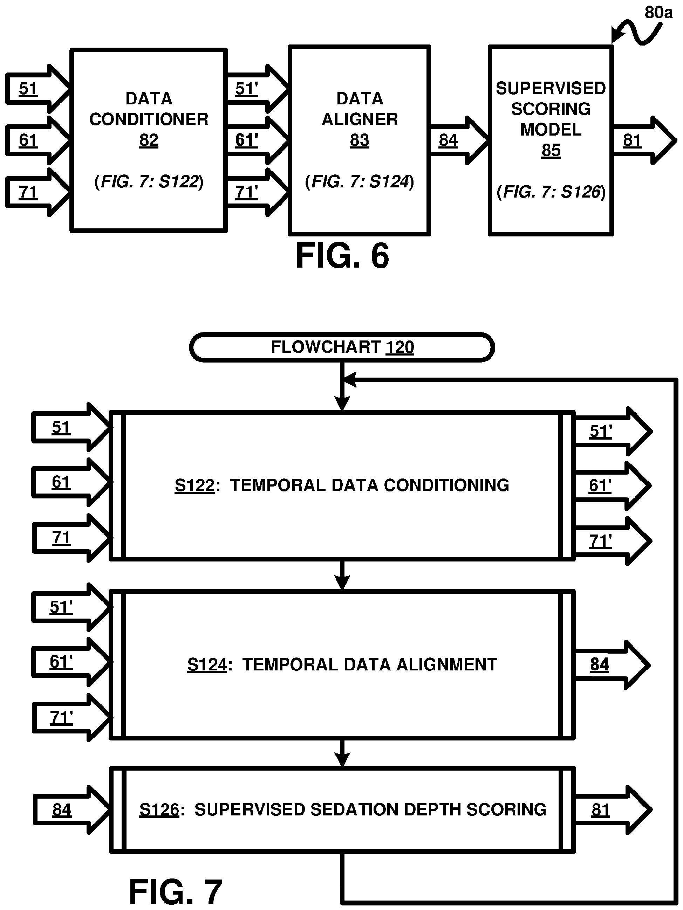

To facilitate a further understanding of the present disclosure, the following description of FIGS. 6 and 7 teaches exemplary embodiments of sedation depth estimator 80 of FIG. 4 and a sedation depth estimation method of FIG. 5 in accordance with the present disclosure. From the description of FIGS. 6 and 7, those having ordinary skill in the art of the present disclosure will appreciate how to apply the present disclosure to make and use additional embodiments of sedation depth estimator and sedation depth estimation methods in accordance with the present disclosure.

FIG. 6 illustrates an embodiment 80a of sedation depth estimator 80 (FIG. 4) employing application modules in the form of a data conditioner 82, a data aggregator 83 and a supervised scoring model 85 for implementing a flowchart 120 of FIG. 7 during stage S112 of flowchart 110 (FIG. 5).

Referring to FIGS. 4 and 5, in practice, a stage S122 of flowchart 120 encompasses data conditioner 82 implementing management and optimization techniques, as known in the art of the present disclosure or hereinafter conceived, for conditioning patient pharmacological data 51, patient physiological data 61 and/or patient psychological data 71 as needed into respective data trendlines patient pharmacological data trendline 51', patient physiological data trendline 61' and patient psychological data trendline 71'.

For example, patient physiological data 61 and/or patient psychological data 71 may be presented in video format or a waveform, respectively, whereby minimal, if any, conditioning of the video or the waveform is needed (e.g., data extraction, amplification, attenuation, filtering, etc.).

By further example, a data structure of patient pharmacological data 51 may be conditioned to a trendline of data arrays.

Still referring to FIGS. 4 and 5, in practice, a stage S124 of flowchart 120 encompasses data aligner 83 implementing data alignment/padding techniques, as known in the art of the present disclosure or hereinafter conceived, for managing a patient sedation dataset 84 including a temporal alignment 84 of patient pharmacological data trendline 51', patient physiological data trendline 61' and patient psychological data trendline 71' generated during stag S122.

In practice, a stage S126 of flowchart 120 encompasses a supervised scoring machine 85 implementing any supervised learning model to functionally map patient sedation dataset 84 to a patient sedation scale for predicting a continuous estimate of patient sedation depth 81.

Examples of a supervised learning models implemented by supervised scoring machine 85 include, but are not limited to, recurrent neural networks or dynamic Bayesian processes.

Examples of a patient sedation scale include, but are not limited to, a Richmond Agitation Sedation Scale (RSSS) and a Ramsey Sedation Scale (RSS).

Stages S122-S124 are sequentially repeated in a loop for each respiratory cycle until patient 20 is extubated or the monitoring/ventilation is temporally suspended.

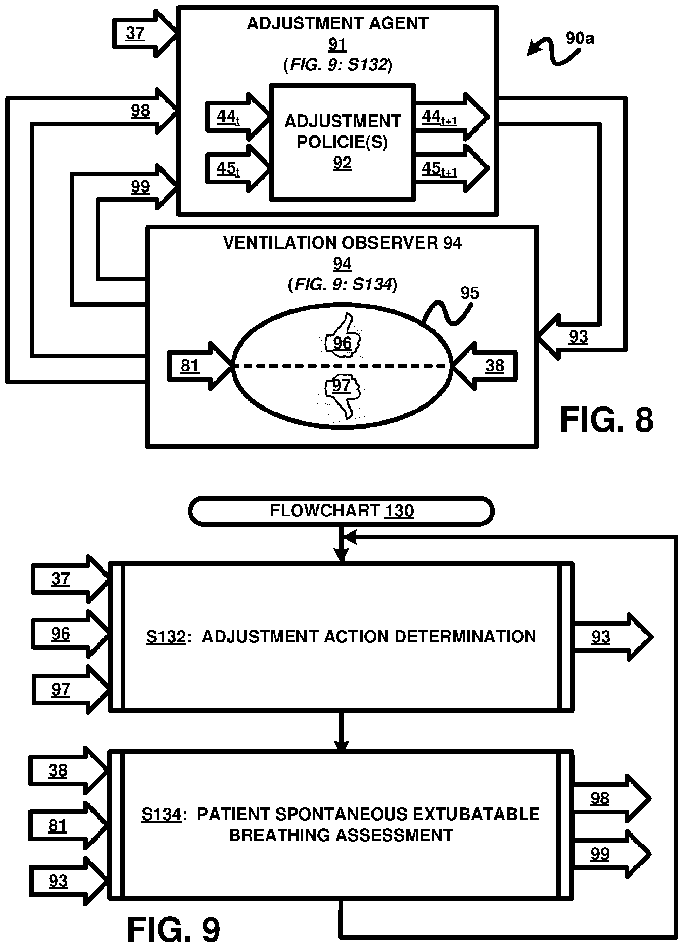

To facilitate a further understanding of the present disclosure, the following description of FIGS. 8 and 9 teaches exemplary embodiments of adaptive ventilation controller 90 of FIG. 4 and an adaptive ventilation adjustment optimization methods in accordance with the present disclosure. From the description of FIGS. 8 and 9, those having ordinary skill in the art of the present disclosure will appreciate how to apply the present disclosure to make and use additional embodiments of adaptive ventilation controllers and adaptive ventilation adjustment optimization methods in accordance with the present disclosure.

FIG. 8 illustrates a reinforcement learning embodiment 90a of adaptive ventilation controller 90 (FIG. 4) employing application modules in the form of adjustment agent 91 and a ventilation observer 94 for implementing a flowchart 140 of FIG. 9 during stage S114 of flowchart 110 (FIG. 5) with a goal to minimize any difference between current respiration patterns of patient 20 and a pattern corresponding to spontaneous breathing by patient 20.

Referring to FIGS. 8 and 9, adjustment agent 91 is trained to know optimal ventilation modes and/or ventilation settings which minimize a duration of patient ventilation. As such, adjustment agent 91 includes adjustment policies 92 formed from prior clinical ventilation knowledge and ventilation protocols, as known in the art of the present disclosure and hereinafter conceived, for determining an adjustment action including a ventilation mode adjustment and/or a ventilation setting adjustment(s). optimal ventilation modes and/or ventilation settings may be constrained by upper and lower bounds for respiration parameters including but not limited to, pulse oxygen saturation, respiration rate, pH and blood gas values as set by prior clinical ventilation knowledge.

Ventilation observer 94 includes an extubation environment 95 formulated from a set of possible patient ventilation responses and a range of estimations of patient sedation depth 81 to thereby develop an environment domain 96 representing actions facilitating an occurrence of an extubation of patient 20, and an environment domain 97 representing actions impeding any occurrence of an extubation of patient 20.

Still referring to FIGS. 8 and 9, in practice, an initial implementation of flowchart 130 relative to a respiratory cycle of patient 20, a stage S132 of flowchart 130 encompasses adjustment agent 91 determining an adjustment action 93 conditionally including ventilation mode adjustment 44 and/or a ventilation setting adjustment(s) 45 from the initial ventilation mode/settings 37 in context of adjustment policies 92.

In turn, a stage S134 of flowchart 130 encompasses ventilation observer 94 processes patient respiration data 38 and estimated patient sedation depth 81 to ascertain if adjustment action 93 increased or decreased an occurrence of an extubation of patient 20. During a current respiratory cycle and prior to a subsequent respiratory cycle, ventilation observer 94 communicates a reward 98 and an extubatable state 99 to adjustment agent 91.

The reward 98 will either be a positive award signaling adjustment agent 91 to continue a planned sequence of actions toward extubation of patient 20, or a negative award signaling adjustment agent 91 to correct a planned sequence of actions toward extubation of patient 20.

The extubatable state 99 informs the adjustment agent whether patient 20 is trending upward towards or downward away from extubation of patient 20 from medical ventilator 40 and may further provide a degree of the strength of the upward trend or a downward trend.

Still referring to FIGS. 8 and 9, in practice, a second iteration of stage S132 involves adjustment agent 91 conditionally revising ventilation mode adjustment 44ₜ₁ and/or a ventilation setting adjustment(s) 45ₜ₁ as needed into a next action ventilation mode adjustment 44ₜ₁₊₁ and/or a ventilation setting adjustment(s) 45ₜ₁₊₁. Any revision of ventilation mode adjustment 44ₜ₁ and/or a ventilation setting adjustment(s) 45ₜ₁ is derived from an application of adjustment policies 92 to reward 98 and extubatable state 99.

Adaptive ventilation controller 90a continues looping through flowchart 130 on a respiration cycle basis until patient 20 is extubated or the monitoring/ventilation is temporally suspended.

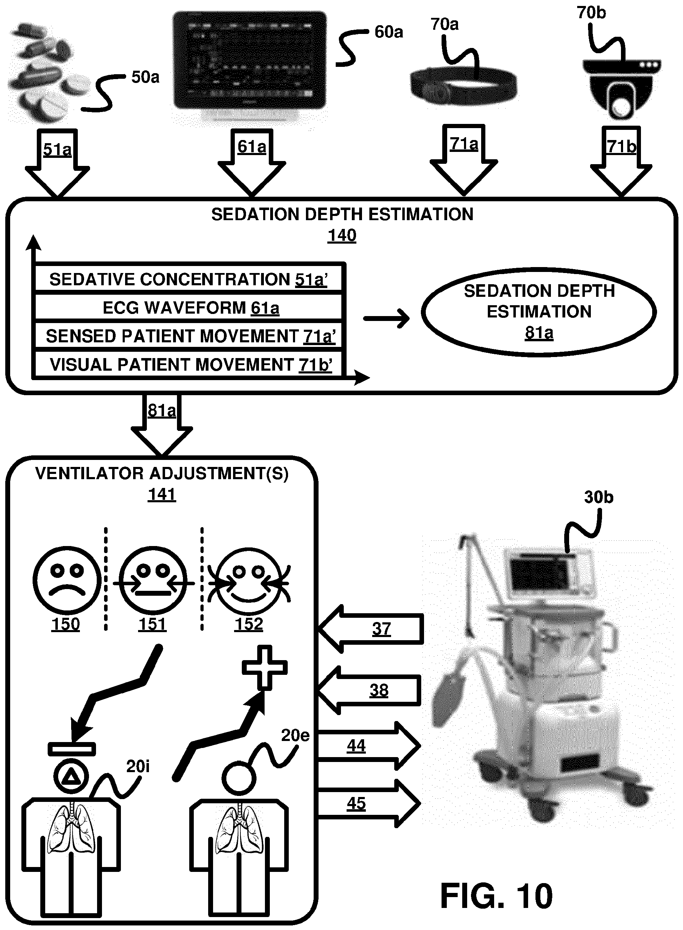

FIG. 10 illustrates exemplary medical ventilation of a patient 20 in accordance with the preceding description of FIGS. 1-9 to thereby provide a simple summary of the present disclosure.

Referring to FIG. 10, a sedation depth estimation stage 140 involves a conditioning and an aligning a data trendline 51a' of pharmacological information 51a on sedative drugs given to patient 20 (e.g., name of drug, dose, time and mode of administration) to estimate the effective sedative concentration in of patient 20 over time, an ECG waveform 61a from an ECG monitor 60a, a data trendline 71a' of a sensing signal from a respiration sensor 70a indicative of torso motion of patient 20, and a data trendline 71b' of a video from a camera 70b illustrative of bodily movements of patient 20 and a sleep/wake cycle of patient 20. The aligned data is used to temporally estimate a patient sedation depth 81a in accordance with present disclosure (e.g., a supervised scoring).

A ventilation adjustment stage 141 involves a processing of patient respiration status 38 and patient sedation depth 81a on a respiration cycle basis in accordance with present disclosure to determine whether patient 20 is negatively trending toward or maintaining an intubation state 20i, or whether patient 20 is positively trending toward or maintaining an extubation state 20e (e.g., a reinforcement learning). To this end, the control of the patient ventilation by medical ventilator 30b may be adapted via ventilation mode adjustment 44 to transition patient 20 between a controlled ventilation mode 150, an assisted ventilation mode 151 and a spontaneous ventilation mode 152. Alternatively or concurrently, the control of the patient ventilation by medical ventilator 30b may be adapted via ventilation setting adjustment(s) 45 to increment or decrement particular ventilation setting(s).

Sedation depth estimation stage 140 ventilation adjustment stage 141 are looped on a respiration cycle basis until patient 20 is extubated or the monitoring/ventilation is temporally suspended.

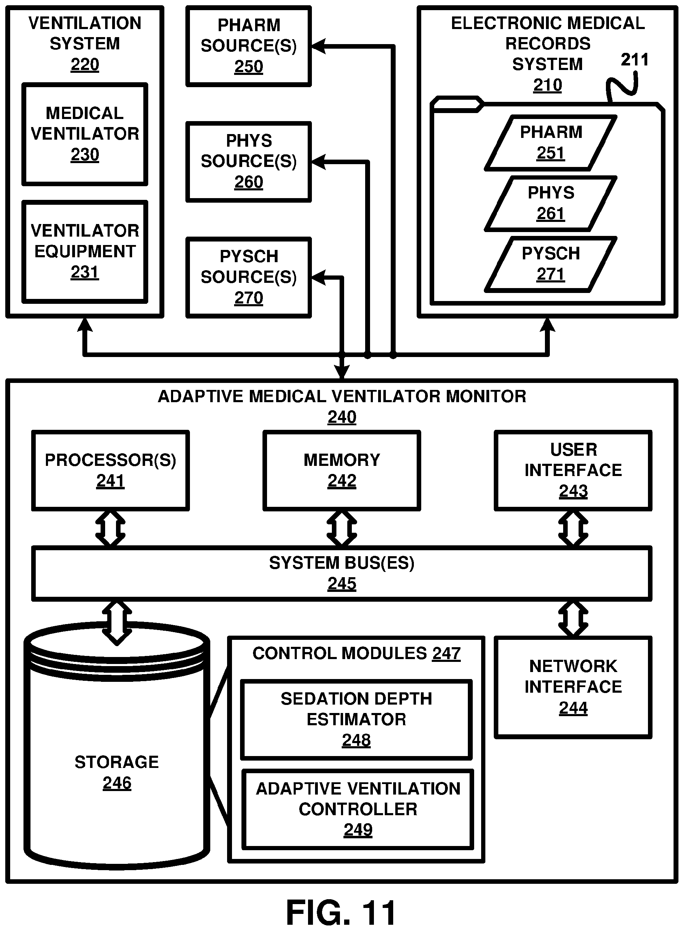

Referring to FIG. 11, an exemplary embodiment 240 of adaptive medical ventilator monitor 40 (FIG. 1) includes one or more processor(s) 241, memory 242, a user interface 243, a network interface 244, and a storage 246 interconnected via one or more system buses 245.

Each processor 241 may be any hardware device, as known in the art of the present disclosure or hereinafter conceived, capable of executing instructions stored in memory 242 or storage or otherwise processing data. In a non-limiting example, the processor(s) 241 may include a microprocessor, field programmable gate array (FPGA), application-specific integrated circuit (ASIC), or other similar devices.

The memory 242 may include various memories, as known in the art of the present disclosure or hereinafter conceived, including, but not limited to, L1, L2, or L3 cache or system memory. In a non-limiting example, the memory 242 may include static random access memory (SRAM), dynamic RAM (DRAM), flash memory, read only memory (ROM), or other similar memory devices.

The user interface 243 may include one or more devices, as known in the art of the present disclosure or hereinafter conceived, for enabling communication with a user such as an administrator. In a non-limiting example, the user interface may include a command line interface or graphical user interface that may be presented to a remote terminal via the network interface 244.

The network interface 244 may include one or more devices, as known in the art of the present disclosure or hereinafter conceived, for enabling communication with ventilation system 220, pharmacological source(s) 250, physiological source(s) 260, psychological source(s) 270 and an electronic medical records system 210. In a non-limiting example, the network interface 244 may include a network interface card (NIC) configured to communicate according to the Ethernet protocol. Additionally, the network interface 244 may implement a TCP/IP stack for communication according to the TCP/IP protocols. Various alternative or additional hardware or configurations for the network interface 244 will be apparent.

The storage 246 may include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 246 may store instructions for execution by the processor(s) 241 or data upon with the processor(s) 241 may operate. For example, the storage 246 may store a base operating system for controlling various basic operations of the hardware. The storage 246 also stores application modules in the form of executable software/firmware for implementing the various functions of adaptive medical ventilator monitor 240 as previously described in the present disclosure including, but not limited to, a sedation depth estimator 248 and an adaptive ventilation controller 249 of the present disclosure.

In practice, adaptive medical ventilator monitor 240 may be a stand-alone monitor, incorporated in ventilation system 220 with medical ventilator 230 and ventilator equipment 231 (e.g., ventilation mask and tubes), or may be incorporated with electronic medical records system 210 with access to a patient sedation record 211.

Referring to FIGS. 1-11, those having ordinary skill in the art of the present disclosure will appreciate numerous benefits of the present disclosure including, but not limited to, an adaptive medical ventilation monitoring via a patient sedation depth for minimizing a ventilation duration of a patient.

Further, as one having ordinary skill in the art will appreciate in view of the teachings provided herein, structures, elements, components, etc. described in the present disclosure/specification and/or depicted in the Figures may be implemented in various combinations of hardware and software, and provide functions which may be combined in a single element or multiple elements. For example, the functions of the various structures, elements, components, etc. shown/illustrated/depicted in the Figures can be provided through the use of dedicated hardware as well as hardware capable of executing software in association with appropriate software for added functionality. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar function, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the various and numerous inventions of the present disclosure (which embodiments are intended to be illustrative and not limiting), it is noted that modifications and variations can be made by persons skilled in the art in light of the teachings provided herein, including the Figures. It is therefore to be understood that changes can be made in/to the preferred and exemplary embodiments of the present disclosure which are within the scope of the embodiments disclosed herein.

Moreover, it is contemplated that corresponding and/or related systems incorporating and/or implementing the device/system or such as may be used/implemented in/with a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. In the device claim enumerating several means, several of these means may be embodied by one and the same item of hardware. Measures recited in mutually different dependent claims may advantageously be used in combination.

## Claims

1. A machine-implemented method for adapting a control of a ventilator (30) to a patient sedation depth associated with patient ventilation and to a patient ventilation response to patient ventilation, the method comprising:
temporally monitoring the patient sedation depth and the patient ventilation response; and
deriving at least one adaptive ventilation adjustment to the control of the ventilator (30) from a cyclic adaptation of the patient ventilation to the temporal monitoring of the patient ventilation and the patient sedation depth.

2. The method of claim 1, wherein the temporally monitoring of the patient sedation depth includes:
temporally estimating the patient sedation depth from a patient sedation dataset including at least one of:
patient pharmacological data informative of a patient sedation profile;
patient physiological data informative of patient biological functioning; and
patient psychological data informative of patient bodily movement.

3. The method of claim 1 or 2, wherein the temporally monitoring of the patient sedation depth includes:
executing a supervised learning model trained on a functional mapping of a patient sedation dataset to a patient sedation scale to render the patient sedation depth;
wherein the patient sedation dataset is informative of at least one of a patient sedation profile, a patient biological functioning and a patient bodily movement; and
wherein the patient sedation scale includes a plurality of patient sedation levels.

4. The method of any of the preceding claims, wherein the deriving the at least one adaptive ventilation adjustment to the control of the ventilator (30) comprises temporally monitoring the patient sedation depth and the patient ventilation response as a basis to minimize a differential between the patient ventilation response and a patient spontaneous breathing.

5. The method of any of the preceding claims, wherein the deriving the at least one adaptive ventilation adjustment to the control of the ventilator (30) comprises executing a reinforcement learning model to temporally observe the patient sedation depth and the patient ventilation response relative to a patient spontaneous breathing.

6. The method of any of the preceding claims, wherein the deriving the at least one adaptive ventilation adjustment to the control of the ventilator (30) comprises:
temporally observing the patient sedation depth and the patient ventilation response relative to a patient spontaneous breathing.

7. A computer program product comprising instructions for a processor to carry out the method of any of the preceding method claims.

8. An adaptive ventilator monitor (40), comprising a processor configured to carry out the method of any of the preceding method claims 1-6.

9. An adaptive ventilation system, comprising:
a ventilator (30) configured to control ventilation; and
an adaptive ventilator monitor (40) as claimed in claim 8.

10. The adaptive ventilation system of claim 9,
wherein the ventilator (30) is configured to control patient ventilation in accordance with a ventilation mode; and
wherein the adaptive ventilator monitor (40) is configured to derive at least one ventilation mode adjustment to the ventilation mode from the cyclic adaptation of the patient ventilation to the temporal monitoring of the patient sedation depth and the patient ventilation response.

11. The adaptive ventilation system of claim 9,
wherein the ventilator (30) is configured to control the patient ventilation in accordance with a ventilation setting; and
wherein the adaptive ventilator monitor (40) is configured to derive at least one ventilation setting adjustment to the ventilation setting from the cyclic adaptation of the patient ventilation to the temporal monitoring of the patient sedation depth and the patient ventilation response.

12. The adaptive ventilation system of any of the preceding claims 9-11,
wherein the ventilator (30) is configured to communicate respiration status data to the adaptive ventilator monitor (40); and
wherein the adaptive ventilator monitor (40) is configured to derive the patient ventilation response from the respiration status data.
